(19) Europäisches Patentamt
European Patent Office
Office européen des brevets

(11) **EP 1 056 800 B1**

(12) **EUROPÄISCHE PATENTSCHRIFT**

(45) Veröffentlichungstag und Bekanntmachung des Hinweises auf die Patenterteilung:
**16.01.2002 Patentblatt 2002/03**

(21) Anmeldenummer: **99910244.5**

(22) Anmeldetag: **19.02.1999**

(51) Int Cl.[7]: **C08J 3/24**, C08K 5/10, C08L 101/14

(86) Internationale Anmeldenummer:
**PCT/EP99/01093**

(87) Internationale Veröffentlichungsnummer:
**WO 99/42515 (26.08.1999 Gazette 1999/34)**

(54) **NACHVERNETZUNG VON HYDROGELEN MITTELS BORSÄUREESTERN**
SECONDARY CROSS-LINKING OF HYDROGELS BY MEANS OF BORIC ACID ESTERS
POST-RETICULATION D'HYDROGELS A L'AIDE D'ESTERS BORIQUES

(84) Benannte Vertragsstaaten:
**BE DE ES FR GB IT NL SE**

(30) Priorität: **21.02.1998 DE 19807501**

(43) Veröffentlichungstag der Anmeldung:
**06.12.2000 Patentblatt 2000/49**

(73) Patentinhaber: **BASF AKTIENGESELLSCHAFT
67056 Ludwigshafen (DE)**

(72) Erfinder:
- **FUNK, Rüdiger
D-65527 Niedernhausen (DE)**
- **FRENZ, Volker
D-55246 Mainz-Kostheim (DE)**
- **STÜVEN, Uwe
D-65812 Bad Soden (DE)**
- **ENGELHARDT, Fritz
Chesapeake, VA 23320 (US)**
- **DANIEL, Thomas
Chesapeake, VA 23321 (US)**

(56) Entgegenhaltungen:
**EP-A2- 0 370 827**
**US-A1- 4 497 930**
**US-A1- 4 587 308**
**US-A1- 4 933 213**
**US-A1- 5 502 082**
**US-A1- 5 662 708**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 056 800 B1

Printed by Jouve, 75001 PARIS (FR)

## Beschreibung

Die vorliegende Erfindung betrifft ein Verfahren zur Gel- bzw. Oberflächennachvernetzung von wasserabsorbierenden Hydrogelen mittels Borsäureestern mehrwertiger Alkohole, die so erhältlichen wasserabsorbierenden Polymeren und ihre Verwendung in Hygieneartikeln und Verpackungsmaterialien.

Hydrophile, hochquellfähige Hydrogele sind insbesondere Polymere aus (co)polymerisierten hydrophilen Monomeren, Pfropf(co)polymere von einem oder mehreren hydrophilen Monomeren auf einer geeigneten Pfropfgrundlage, verneczte Cellulose- oder Stärkeether, vernetzte Carboxymethylcellulose, teilweise vernetztes Polyalkylenoxid oder in wäßrigen Flüssigkeiten quellbare Naturprodukte, wie beispielsweise Guarderivate. Solche Hydrogele werden als wäßrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Zur Verbesserung der Anwendungseigenschaften, wie z.B. Rewet in der Windel und AUL (Absorption unter Belastung), werden hydrophile, hochquellfähige Hydrogele im allgemeinen Oberflächen- oder gelnachvernetzt. Diese Nachvernetzung ist dem Fachmann an sich bekannt und erfolgt bevorzugt in wäßriger Gelphase oder als Oberflächennachvernetzung der gemahlenen und abgesiebten Polymerpartikel.

Dazu geeignete Vernetzer sind Verbindungen, die mindestens zwei Gruppen enthalten, die mit den Carboxylgruppen des hydrophilen Polymeren kovalente Bindungen bilden können. Geeignete Vernetzer sind beispielsweise Di- oder Polyglycidylverbindungen, wie Phosphonsäurediglycidylester, Alkoxysilylverbindungen, Polyaziridine, Polyamine oder Polyamidoamine, wobei die genannten Verbindungen auch in Mischungen untereinander verwendet werden können (siehe beispielsweise EP-A-0 083 022, EP-A-0 543 303 und EP-A-0 530 438). Als Vernetzer geeignete Polyamidoamine sind insbesondere in der EP-A-0 349 935 beschrieben.

Ein wesentlicher Nachteil dieser Vernetzer ist deren hohe Reaktivität. Diese ist zwar, was den chemischen Umsatz betrifft, erwunscht, birgt aber ein hoheres toxikologisches Potential. Die Verarbeitung derartiger Vernetzer im Produktionsbetrieb erfordert besondere Schutzvorkehrungen, um den Anforderungen der geltenden Sicherheitsbestimmungen und der Arbeitsplatzhygiene gerecht zu werden. Darüber hinaus erscheint die Verwendung derartig modifizierter Polymere in Hygieneartikeln bedenklich.

Als Vernetzer sind auch polyfunktionelle Alkohole bekannt. Beispielsweise lehren EP-A-0 372 981, US-A-4 666 983 sowie US-A-5 385 983 die Verwendung von hydrophilen Polyalkoholen bzw. die Verwendung von Polyhydroxytensiden. Die Reaktion wird hiernach bei Temperaturen von 120-250°C durchgeführt. Das Verfahren hat den Nachteil, daß die zur Vernetzung führende Veresterungsreaktion selbst bei diesen Temperaturen nur relativ langsam abläuft.

Somit bestand die Aufgabe darin, unter Verwendung relativ reaktionsträger, aber dennoch mit Carboxylgruppen reaktionsfähiger Verbindungen eine im Vergleich zum Stand der Technik ebenso gute oder bessere Gel- bzw. Oberflächennachvernetzung zu erreichen. Diese Aufgabe war so zu lösen, daß die Reaktionszeit möglichst kurz und die Reaktionstemperatur möglichst niedrig sind. Im Idealfall sollten dieselben Reaktionsbedingungen herrschen wie bei der Verwendung von hochreaktiven Epoxiden.

Überraschend wurde nun gefunden, daß Ester der Borsäure mit mehrwertigen Alkoholen als Oberflächennachvernetzungsmittel gut geeignet sind. Diese Ester sind synthetisch leicht zugänglich durch Umsetzung von Borsäure oder Boroxid mit Alkohol.

Gegenstand der Erfindung ist ein Verfahren zur Oberflächennachvernetzung wasserabsorbierender Polymere durch Behandlung der Polymeren mit einer Oberflächennachvernetzungslösung, wobei die Polymeren während oder nach dem Behandeln durch Temperaturerhöhung nachvernetzt und getrocknet werden, dadurch gekennzeichnet, daß der Vernetzer einen Ester der Borsäure mit einem zwei- oder mehrwertigen Alkohol in einem inerten Lösemittel gelöst enthält.

Unter einem Borsäureester versteht man eine Verbindung der Formel $B(OR)_3$. Borsäureester bilden sich z. B. bei der Reaktion von Borsäureanhydrid $B_2O_3$ mit Alkoholen unter gleichzeitiger Bildung von Borsäure gemäß

$$B_2O_3 + 3\ ROH \rightarrow B(OR)_3 + H_3BO_3$$

oder bei höherem Alkoholüberschuß gemäß

$$B_2O_3 + 6\ ROH \rightarrow 2\ B(OR)_3 + 3\ H_2O$$

oder durch die Reaktion von Borsäure mit Alkoholen bei gleichzeitiger Abtrennung des Wasser während der Veresterungsreaktion gemäß

$$B(OH)_3 + 3\ ROH \rightarrow B(OR)_3 + 3\ H_2O$$

Höhere Ester der Borsäure sind beispielsweise durch Umesterungsreaktionen zugänglich:

$$B(OR^1)_3 + 3\ R^2OH \rightarrow B(OR^2)_3 + 3\ R^1OH,$$

wobei der niedrigsiedendere Alkohol $R^1OH$ destillativ aus der Gemisch getrennt wird.

Bei den im erfindungsgemäßen Verfahren zur Oberflächennachvernetzung eingesetzten Borsäureester handelt es sich um Ester di- oder polyfunktioneller Alkohole. Bei Umsetzung der Borsaure oder des Borsäureanhydrids oder bei der Umesterungsreaktion mit bi- oder polyfunktionellen Alkoholen können sich auch cyclische Verbindungen oder Polyester bilden. Betrachtet man die Reaktion von Ethylenglykol ($R^1$=H in den Formeln 1a-1d) oder Propandiol-1,2 ($R^1$= $CH_3$ in den Formeln 1a-1d), so können sich folgende Borsäureester bilden:

Bei einem stöchiometrischen Unterschuß von Alkohol bildet sich zunächst bevorzugt eine teilveresterte Borsäure wie z.B.

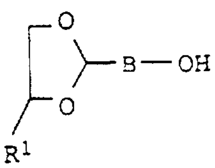

(1a)

oder auch das entsprechende Anhydrid dieser Verbindungen:

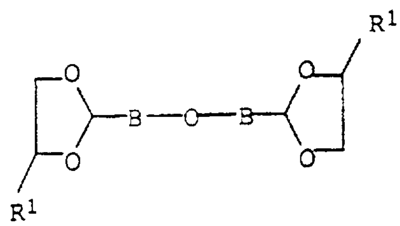

(1b)

Eine vollständige Veresterung führt bevorzugt zu folgenden Produkten:

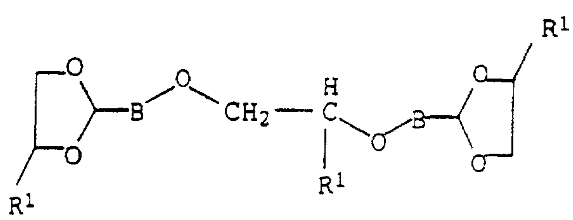

(1c)

daneben in geringerem Ausmaß zu zyklischen Verbindungen und Polyester mit folgender Wiederholungseinheit

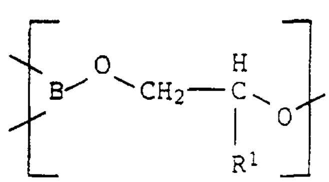

(1d)

Verwendet man andere, von Ethylenglykol oder Propandiol-1,2 verschiedene di- oder polyfunktionelle Alkohole, so bilden sich die analogen Borsäureester.

Der Rest $R^1$ steht für Wasserstoff oder eine Alkylgruppe, vorzugsweise mit 1 bis 12, insbesondere 1 bis 6 Kohlenstoffatomen.

Die Temperatur zur Nachvernetzung beträgt bevorzugt 50-250°C, insbesondere zwischen 50-200°C, speziell zwischen 100-180°C.

Zur Beschleunigung der Reaktion der Oberflächennachvernetzungslösung kann ein saurer Katalysator zugesetzt werden. Als Katalysator im erfindungsgemäßen Verfahren sind alle anorganischen Säuren, deren korrespondierende Anhydride, bzw. organischen Säuren und deren korrespondierende Anhydride verwendbar. Beispiele sind Borsäure, Schwefelsäure, Iodwasserstoffsäure, Phosphorsäure, Weinsäure, Essigsäure und Toluolsulfonsäure. Insbesondere sind auch deren polymere Formen, Anhydride, sowie die sauren Salze der mehrwertigen Säuren geeignet. Beispiele hierfür sind Boroxid, Schwefeltrioxid, Diphosphorpentoxid und Ammoniumdihydrogenphosphat.

Das erfindungsgemäße Verfahren wird vorzugsweise so durchgeführt, daß eine Lösung des Oberflächennachvernetzers auf das trockene Grundpolymerpulver aufgesprüht wird. Im Anschluß an das Aufsprühen wird das Polymerpulver thermisch getrocknet, wobei die Vernetzungsreaktion sowohl vor als auch während der Trocknung stattfinden kann. Bevorzugt ist das Aufsorühen einer Lösung des Vernetzers in Reaktionsmischern und Spruhmischern oder Misch- und Trocknungsanlagen wie beispielsweise Lödige-Mischer, ®BEPEX-Mischer, ®NAUTA-Mischer, ®SHUGGI-Mischer oder ®PROCESSALL. Überdies können auch Wirbelschichttrockner eingesetzt werden. Die Trocknung kann im Mischer selbst erfolgen, durch Beheizung des Mantels oder Einblasen von Warmluft. Ebenso geeignet ist ein nachgeschalteter Trockner wie z.B. ein Hordentrockner, ein Drehrohrofen, oder eine beheizbare Schnecke. Es kann aber auch beispielsweise eine azeotrope Destillation als Trocknungsverfahren benutzt werden. Die Verweilzeit bei der bevorzugten Temperatur im Reaktionsmischer oder Trockner liegt bei 5 bis 90 Minuten, bevorzugt bei weniger als 30 Minuten, ganz besonders bevorzugt bei weniger als 10 Minuten.

Als inertes Lösemittel setzt man bevorzugt Wasser sowie Gemische von Wasser mit einwertigen oder mehrwertigen Alkoholen ein. Es können jedoch alle mit Wasser unbegrenzt mischbaren organischen Lösemittel, wie beispielsweise bestimmte Ester und Ketone eingesetzt werden, die nicht selbst unter den Verfahrensbedingungen reaktiv sind. Sofern ein Alkohol/Wasser-Gemisch eingesetzt wird, beträgt der Alkoholgehalt dieser Lösung beispielsweise 10-90 Gew.-%, bevorzugt 30-70 Gew.-%, insbesondere 40-60 Gew.-%. Es können alle mit Wasser unbeschränkt mischbaren Alkohole eingesetzt werden sowie Gemische mehrerer Alkohole (z.B. Methanol + Glycerin + Wasser). Besonders bevorzugt ist die Verwendung folgender Alkohole in wäßriger Lösung: Methanol, Ethanol, Isopropanol, Ethylenglykol und besonders bevorzugt 1,2-Propandiol sowie 1,3-Propandiol. Die Oberflächennachvernetzungslösung wird in einem Verhältnis von 1-20 Gew.-%, bezogen auf die Polymermasse, eingesetzt. Besonders bevorzugt ist eine Lösungsmenge von 2,5-15 Gew.-% bezüglich Polymer. Der Vernetzer selbst wird dabei in einer Menge von 0,01-1,0 Gew.-%, bezogen auf das eingesetzte Polymer, verwendet.

Das wasserabsorbierende Polymer ist bevorzugt eine polymere Acrylsäure oder ein Polyacrylat. Die Herstellung dieses wasserabsorbierenden Polymeren kann nach einem aus der Literatur bekannten Verfahren erfolgen. Bevorzugt sind Polymere, die vernetzende Comonomere enthalten (0,001-10 mol-%), ganz besonders bevorzugt sind jedoch Polymere die über radikalische Polymerisation erhalten wurden und bei denen ein mehrfunktioneller ethyienisch ungesättigter Radikalvernetzer verwendet wurde, der zusätzlich noch mindestens eine freie Hydroxylgruppe trägt (wie z.B. Pentaerythritoltriallylether oder Trimethylolpropandiallylether).

Die im erfindungsgemäßen Verfahren einzusetzenden hydrophilen, hochquellfähigen Hydrogele sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether oder in wäßrigen Flüssigkeiten quellbare Naturprodukte, wie beispielsweise Guarderivate. Diese Hydrogele sind dem Fachmann bekannt und beispielsweise beschrieben in US-A-4 286 082, DE-C-27 06 135, US-A-4 340 706, DE-C-37 13 601, DE-C-28 40 010, DE-A-43 44 548, DE-A-40 20 780, DE-A-40 15 085, DE-A-39 17 846, DE-A-38 07 289, DE-A-35 33 337, DE-A-35 03 458, DE-A-42 44 548, DE-A-42 19 607, DE-A-40 21 847, DE-A-38 31 261, DE-A-35 11 086, DE-A-31 18 172, DE-A-30 28 043, DE-A-44 18 881, EP-A-0 801 483, EP-A-0 455 985, EP-A-0 467 073, EP-A-0 312 952, EP-A-0 205 874, EP-A-0 499 774, DE-A-26 12 846, DE-A-40 20 780 EP-A-0 205 674, US-5 145 906, EP-A-0 530 438, EP-A-0 670 073, US-A-4 057 521, US-A-4 062 817, US-A-4 525 527, US-A-4 295 987, US-A-5 011 892, US-A-4 076 663 oder US-A-4 931 497. Der Inhalt der vorstehend genannten Patentdokumente ist ausdrücklich Bestandteil der vorliegenden Offenbarung.

Zur Herstellung dieser hydrophilen, hochquellfähigen Hydrogele geeignete hydrophile Monomere sind beispielsweise polymerisationsfähige Säuren, wie Acrylsäure, Methacrylsäure, Vinylsulfonsäure, Vinylphosphonsäure, Maleinsäure einschließlich deren Anhydrid, Fumarsäure, Itaconsäure, 2-Acrylamido-2-methylpropansulfonsäure, 2-Acrylamido-2-methylpropanphosphonsäure, Alkalimetall- und Ammoniumsalze der Säuregruppen enthaltenden Monomeren, sowie deren Amide, Hydroxyalkylester und Aminoalkylgruppen- oder ammoniumgruppenhaltige Ester und Amide. Des weiteren sind wasserlösliche N-Vinylamide wie N-Vinylformamid oder auch Diallyldimethylammoniumchlorid geeignet. Bevorzugte hydrophile Monomere sind Verbindungen der Formel

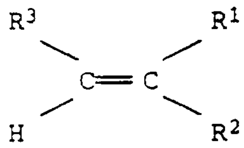

(2)

worin

$R^1$ Wasserstoff, Methyl oder Ethyl,

$R^2$ $-COOR^4$, eine Sulfonylgruppe, eine Phosphonylgruppe, eine mit $(C_1-C_4)$-Alkanol veresterte Phosphonylgruppe oder eine Gruppe der Formel

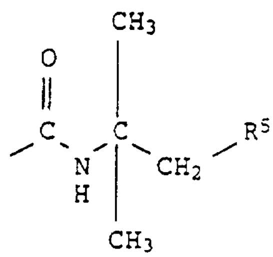

(3),

$R^3$ Wasserstoff, Methyl, Ethyl oder eine Carboxylgruppe,

$R^4$ Wasserstoff, Alkalimetall- oder Ammoniumion, Amino-$(C_1-C_4)$-Alkyl oder Hydroxy-$(C_1-C_4)$-Alkyl und

$R^5$ eine Sulfonylgruppe, eine Phosphonylgruppe, eine Carboxylgruppe oder die Alkalimetall- oder Ammoniumsalze dieser Gruppen bedeuten.

Beispiele für $(C_1-C_4)$-Alkanole sind Methanol, Ethanol, n-Propanol, Isopropanol oder n-Butanol. Besonders bevorzugte hydrophile Monomere sind Acrylsäure und Methacrylsäure, sowie die Natrium-, Kalium- und Ammoniumsalze dieser Säuren. Gegebenenfalls können diese Säuren auch in teilweise neutralisierter Form vorliegen.

Geeignete Pfropfgrundlagen für hydrophile Hydrogele, die durch Pfropfcopolymerisation olefinisch ungesättigter Säuren erhältlich sind, können natürlichen oder synthetischen Ursprungs sein. Beispiele sind Stärke, Cellulose oder Cellulosederivate sowie andere Polysaccharide und Oligosaccharide, Polyalkylenoxide, insbesondere Polyethylenoxide und Polypropylenoxide, sowie hydrophile Polyester.

Geeignete Polyalkylenoxide haben beispielsweise die Formel

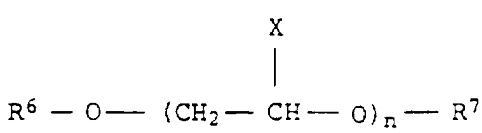

(4)

worin

$R^6$ und $R^7$ unabhängig voneinander Wasserstoff, Alkyl, Alkenyl oder Acyl,

X Wasserstoff oder Methyl und

n eine ganze Zahl von 1 bis 10 000 bedeuten.

$R^6$ und $R^7$ bedeuten bevorzugt Wasserstoff, $(C_1-C_4)$-Alkyl, $(C_2-C_6)$-Alkenyl oder Phenyl. Bevorzugte Hydrogeie

sind insbesondere Polyacrylate, Polymethacrylate sowie die in US-A-4 931 497, US-A-5 011 892 und US-A-5 041 496 beschriebene Pfropfcopolymere.

Die hydrophilen, hochquellfähigen Hydrogele sind bevorzugt vernetzt, d.h. sie enthalten Verbindungen mit mindestens zwei Doppelbindungen, die in das Polymernetzwerk einpolymerisiert sind. Geeignete Vernetzer sind insbesondere Methylenbisacryl-bzw. -methacrylamid, Ester ungesättigter Mono- oder Polycarbonsäuren von Polyolen, wie Diacrylat oder Triacrylat, z.B. Butandiol- oder Ethylenglykoldiacrylat bzw. -methacrylat sowie Trimethylolpropantriacrylat und Allylverbindungen wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in der EP-A-0 343 427 beschrieben sind. Besonders bevorzugt werden im erfindungsgemäßen Verfahren jedoch Hydrogele, die unter Verwendung von Polyallylethern als Vernetzer und durch saure Homopolymerisation von Acrylsäure hergestellt werden. Geeignete Vernetzer sind Pentaerythritoltri- und -tetraallylether, Polyethylenglykoldiallylether, Monoethylenglykoldiallylether, Glyceroldi- und triallylether, Polyallylether auf Basis Sorbitol, sowie alkoxylierte Varianten davon.

Die hydrophilen, hochquellfähigen Hydrogele können durch an sich bekannte Polymerisationsverfahren hergestellt werden. Bevorzugt ist die Polymerisation in wäßriger Lösung nach dem Verfahren der sogenannten Gelpolymerisation. Dabei werden 15 bis 50 gew.-%ige wäßrige Lösungen eines oder mehrerer hydrophiler Monomerer und gegebenenfalls einer geeigneten Pfropfgrundlsge in Gegenwart eines Radikalinitiators, vorzugsweise ohne mechanische Durchmischung, unter Ausnutzung des Trommsdorff-Norrish-Effektes (Makromol. Chem. 1, 169 (1947)), polymerisiert.

Die Polymerisationsreaktion kann im Temperaturbereich zwischen 0°C und 150°C, vorzugsweise zwischen 10°C und 100°C, sowohl bei Normaldruck als auch unter erhöhtem oder erniedrigtem Druck durchgeführt werden. Die Polymerisation kann auch in einer Schutzgasatmosphäre, vorzugsweise unter Stickstoff, ausgeführt werden.

Zur Auslösung der Polymerisation können energiereiche elektromagnetische Strahlen oder die üblichen chemischen Polymerisationsinitiatoren herangezogen werden. Solche sind beispielsweise organische Peroxide, wie Benzoylperoxid, tert. Butylhydroperoxid, Methylethylketonperoxid, Cumolhydroperoxid, Azoverbindungen wie Azodiisobutyronitril sowie anorganische Peroxiverbindungen wie $(NH_4)_2S_2O_8$, $K_2S_2O_8$ oder $H_2O_2$. Sie können gegebenenfalls in Kombination mit Reduktionsmitteln wie Natriumhydrogensulfit, Eisen(II)-sulfat oder Redoxsystemen verwendet werden. Redoxsysteme enthalten als reduzierende Komponente im allgemeinen eine aliphatische und aromatische Sulfinsäure, wie Benzolsulfinsäure oder Toluolsulfinsäure oder Derivate dieser Säuren enthalten, wie z.B. Mannichaddukte aus Sulfinsäure, Aldehyden und Aminoverbindungen, wie sie in DE-C-13 01 566 beschrieben sind.

Durch mehrstündiges Nachheizen der Polymerisatgele im Temperaturbereich 50 bis 130°C, vorzugsweise 70 bis 100°C, können die Qualitätseigenschaften der Polymerisate noch verbessert werden.

Die erhaltenen Gele werden zu 0 - 100 mol-%, bezogen auf eingesetztes Monomer, neutralisiert, bevorzugt zu 25 - 100 mol-%, und besonders bevorzugt zu 50 - 85 mol-%, wobei die üblichen Neutralisationsmittel verwendet werden können, bevorzugt Alkalimetallhydroxide oder -oxide, besonders bevorzugt jedoch Natriumhydroxid, Natriumcarbonat oder Natriumhydrogencarbonat. Üblicherweise wird die Neutralisation durch Einmischung des Neutralisationsmittels als wäßrige Lösung oder bevorzugt auch als Feststoff erreicht. Das Gel wird hierzu mechanisch zerkleinert, beispielsweise mittels eines Fleischwolfes, und das Neutralisationsmittel wird aufgesprüht, übergestreut oder aufgegossen, und dann sorgfältig untergemischt. Dazu kann die erhaltene Gelmasse noch mehrmals zur Homogenisierung gewolft werden.

Die neutralisierte Gelmasse wird dann mit einem Band- oder Walzentrockner getrocknet bis der Restfeuchtegehalt unter 10 Gew.-%, bevorzugt unter 5 Gew.-% liegt. Das getrocknete Hydrogel wird hernach gemahlen und gesiebt, wobei zur Mahlung üblicherweise Walzenstühle, Stiftmühlen oder Schwingmühlen eingesetzt werden können. Die bevorzugte Partikelgröße des gesiebten Hydrogels liegt im Bereich 45-1000 µm, besonders bevorzugt bei 45-850 µm, und ganz besonders bevorzugt bei 200-850 µm.

Ein weiterer Gegenstand der Erfindung ist ein wasserabsorbierendes Polymer, das nach dem oben beschriebenen Verfahren erhältlich ist.

Ein weiterer Gegenstand der Erfindung ist die Verwendung der nach dem erfindungsgemäßen Verfahren hergestellten Produkte in Hygenieartikeln, Verpackungsmaterialien und in Nonwovens.

Zur Bestimmung der Güte der Oberflächennachvernetzung wird das getrocknete Hydrogel dann mit den im Stand der Technik bekannten, Testmethoden geprüft, die nachfolgend beschrieben sind:

Methoden:

1) Zentrifugenretentionskapazität (CRC):

Bei dieser Methode wird die freie Quellbarkeit des Hydrogels im Teebeutel bestimmt. Es werden ca. 0.200 g

trockenes Hydrogel in einen Teebeutel eingeschweißt (Format: 60 mm x 60 mm, Dexter 1234T-Papier) und für 30 Minuten in einer 0,9 gew.-%ige Kochsalzlösung eingeweicht. Anschließend wird der Teebeutel 3 min in einer handelsüblichen Wäschezentrifuge (Bauknecht WS 130, 1400 U/ min, Korbdurchmesser 230 mm) geschleudert. Die Bestimmung der aufgenommenen Flüssigkeitsmenge geschieht durch auswägen des zentrifugierten Teebeutels. Zur Berücksichtigung der Aufnahmekapazität des Teebeutels selbst wird ein Blindwert bestimmt (Teebeutel ohne Hydrogel), welcher von der Auswaage (Teebeutel mit gequollenem Hydrogel) abgezogen wird.

$$\text{Retention CRC [g/g]} = (\text{Auswaage Teebeutel} - \text{Blindwert} - \text{Einwaage Hydrogel}) \div \text{Einwaage Hydrogel}$$

2) Absorption unter Druck (2068,5 Pa (0,3 psi)/3447,5 Pa (0,5 psi)/4826,5 Pa(0,7psi):

Bei der Absorption unter Druck werden 0,900 g trockenen Hydrogels gleichmäßig auf dem Siebboden einer Meßzelle verteilt. Die Meßzelle besteht aus einem Plexiglaszylinder (Höhe = 50 mm, Durchmesser = 60 mm), auf den als Boden ein Sieb aus Stahlgewebe (Maschenweite 36 micron bzw. 400 mesh) aufgeklebt ist. Über das gleichmäßig verteilte Hydrogel wird eine Abdeckplatte gelegt und mit einem entsprechenden Gewicht belastet. Die Zelle wird dann auf ein Filterpapier (S&S 589 Schwarzband, Durchmesser = 90 mm) gestellt, welches auf einer porösen Glasfilterplatte liegt, diese Filterplatte liegt in einer Petrischale (Höhe = 30 mm, Durchmesser = 200 mm), welche soviel 0,9 gew.-%ige Kochsalzlösung enthält, daß der Flüssigkeitsspiegel zu Beginn des Experiments identisch mit der Oberkante der Glasfritte ist. Man läßt das Hydrogel dann für 60 min die Salzlösung absorbieren. Dann nimmt man die komplette Zelle mit dem gequollenen Gel von der Filterplatte, und wägt die Apparatur nach Entfernen des Gewichts zurück.

Die Absorption unter Druck (AUL = Absorbency under load) wird wie folgt berechnet:

$$\text{AUL [g/g]} = ( Wb - Wa ) / WS$$

wobei Wb die Masse der Apparatur + Gel nach dem Quellen ist, Wa die Masse der Apparatur + Einwaage vor dem Quellen ist, Ws die Einwaage an trockenem Hydrogel ist.

Die Apparatur besteht aus Messzylinder und Abdeckplatte.

Beispiele

Herstellung von Borsäureestern

Borsäureester 1

In einem Dreihalskolben mit Rührer, Innenthermometer und Rückflußkühler werden 9 mol Ethylenglykol vorgelegt und 1 mol Borsäureanhydrid langsam zugegeben. Die Lösung wird bei 80°C 2 Stunden gerührt. Danach werden nicht umgesetztes Ethylenglykol und Wasser bei reduziertem Druck destillativ abgetrennt. Beim Abkühlen bildet sich eine weiße, wachsartige Substanz.

Borsäureester 2

In einem Dreihalskolben mit Rührer, Innenthermometer und Rückflußkühler werden 4 mol Propandiol (1,2) vorgelegt und 1 mol Borsäure zugegeben. Es wird bis zum Sieden erhitzt und das Wasser bei Normaldruck abdestilliert. Danach wird bei reduziertem.Druck das überschüssige Propandiol-1,2 abdestilliert. Man erhält ebenfalls eine beim Abkühlen wachsartige erstarrende, weiße Substanz.

Diese Borsäureester werden erfindungsgemäß zur Vernetzung von superabsorbierenden Polymeren eingesetzt. Die folgenden Beispiele verdeutlichen dabei die vernetzende Wirkung der Borsäureester.

Beispiel 1

In einem 40 1-Plastikeimer werden 6,9 kg reine Acrylsäure mit 23 kg Wasser verdünnt. Zu dieser Lösung fügt man 45 g Pentaerythritoltriallylether unter Rühren hinzu, und inertisiert den verschlossenen Eimer durch Durchleiten von Stickstoff. Die Polymerisation wird dann durch Zugabe von ca. 400 mg Wasserstoffperoxid und 200 mg Ascorbinsäure gestartet. Nach Beendung der Reaktion wird das Gel mechanisch zerkleinert, und mit soviel Natronlauge versetzt

bis ein Neutralisationsgrad von 75 mol-% bezogen auf die eingesetzte Acrylsäure erreicht wird. Das neutralisierte Gel wird dann auf einem Walzentrockner getrocknet, mit einer Stiftmühle gemahlen, und schließlich abgesiebt. Dies ist das in den nachfolgenden Beispielen verwendete Grundpolymer.

Dieses Grundpolymer (1 kg) wird im Loedige-Pflugscharmischer in einem 2-Stufenprozeß mit der Oberflächennachvernetzungslösung besprüht.

Stufe 1: Zunächst wird eine Lösung des Borsäureesters 1 (0,5 Gew.-%, bezogen auf Grundpolymer) in dem Lösungsmittel Ethylenglykol (5 Gew.%, bezogen auf Grundpolymer) aufgesprüht.

Stufe 2: Anschließend wird die Temperatur des Heizmantels linear von 50°C auf 200°C gesteigert. Sobald die Produkttemperatur 80-90°C erreicht hat, wird zusätzlich 5 Gew.-% Wasser (bezogen auf Grundpolymer) aufgesprüht. Nach ca. 30 min ist der Prozess beendet, das Hydrogel wird nochmals gesiebt, um Klumpen zu entfernen und kann dann z.B. als wasserabsorbierendes Polymer in Windeln eingesetzt werden. Die Meßwerte für CRC und AUL sind in der Tabelle angegeben.

Beispiel 2

Ein gemäß Beispiel 1 hergestelltes Grundpolymer wird in einem Waring-Labormischer mit Vernetzer-Lösung besprüht. Die Lösung ist dabei so zusammengesetzt, daß - bezogen auf eingesetztes Grundpolymer - folgende Dosierung erreicht wird: 0,5 Gew.-% Borsäureester 1, 4,5 Gew.-% Propylenglykol und 4,5 Gew.-% Wasser. Das feuchte Polymer wird dann bei 175°C für 60 min getrocknet. In der Tabelle sind die Meßwerte für CRC und AUL angegeben.

Beispiel 3

Ein gemäß Beispiel 1 hergestelltes Grundpolymer wird in einem Waring-Labormischer mit Vernetzer-Lösung besprüht. Die Lösung ist dabei so zusammengesetzt, daß - bezogen auf eingesetztes Grundpolymer - folgende Dosierung erreicht wird: 0,5 Gew.-% Borsäureester 2, 4,5 Gew.-% Propylenglykol und 4,5 Gew.-% Wasser. Das feuchte Polymer wird dann bei 175°C für 60 min getrocknet. Die typischen Eigenschaften des Polymers sind in der Tabelle angegeben.

Beispiel 4

Ein gemäß Beispiel 1 hergestelltes Grundpolymer wird in einem Telschig-Labormischer mit Vernetzer-Lösung besprüht. Die Lösung ist dabei so zusammengesetzt, daß - bezogen auf eingesetztes Grundpolymer - folgende Dosierung erreicht wird: 0,5 Gew-.% Borsäureester 2, 7 Gew.-% Methanol und 3 Gew.-% Wasser. Das feuchte Polymer wird dann bei 150°C für 60 min getrocknet. Die typischen Eigenschaften dieses wasserabsorbierenden Polymers sind in der Tabelle angegeben.

Tabelle

| Polymer gemäß | CRC [g/g] | AUL 2068,5 Pa (0.3 psi) [g/g] | AUL 3447,5 Pa (0.5 psi) [g/g] | AUL 4826,5 Pa (0.7 psi) [g/g] |
|---|---|---|---|---|
| Beispiel 1 | 35 | 35 | 26 | 18 |
| Beispiel 2 | 37 | 34 | 20 | 14 |
| Beispiel 3 | 33 | 32 | 28 | 23 |
| Beispiel 4 | 29 | 30 | 27 | 25 |
| Vergleich Grundpolymer | 42 | 10 | 9 | 9 |

## Patentansprüche

1. Verfahren zur Oberflächennachvernetzung wasserabsorbierender Polymere durch Behandlung der Polymeren mit einer Oberflächennachvernetzungslösung, wobei die Polymeren während oder nach dem Behandeln durch Temperaturerhöhung nachvernetzt und getrocknet werden, **dadurch gekennzeichnet, daß** der Vernetzer einen Ester der Borsäure mit einem zwei- oder mehrwertigen Alkohol in einem inerten Lösemittel gelöst enthält.

2. Verfahren nach Anspruch 1, **dadurch gekennzeichnet, daß** das wasserabsorbierende Polymer eine polymere

Acrylsäure oder ein Polyacrylat ist, insbesondere eine polymere Acrylsäure oder ein Polyacrylat, die durch radikalische Polymerisation in Gegenwart eines mehrfunktionellen ethylenisch ungesättigten Radikalvernetzers, der zusätzlich noch eine oder mehrere freie Hydroxylgruppe tragen kann, erhalten wurden.

**3.** Verfahren nach Anspruch 1 und/oder 2, **dadurch gekennzeichnet, daß** zur Vernetzung ein Katalysator verwendet wird, der eine anorganische Säure, deren korrespondierendes Anhydrid, eine organische Säure oder deren korrespondierendes Anhydrid umfaßt.

**4.** Verfahren nach Anspruch 3, **dadurch gekennzeichnet, daß** es sich bei den Säuren um Borsäure, Schwefelsäure, Iodwasserstoffsäure, Phosphorsäure, Weinsäure, Essigsäure, Toluolsuifonsäure, sowie deren polymere Formen, Anhydride oder sauren Salze handelt.

**5.** Verfahren nach einem oder mehreren der Ansprüche 1 bis 4, **dadurch gekennzeichnet, daß** das inerte Lösemittel Wasser, ein Gemisch von Wasser mit in Wasser unbegrenzt löslichen organischen Lösemitteln oder ein Gemisch von Wasser mit einwertigen oder mehrwertigen Alkoholen ist.

**6.** Verfahren nach Anspruch 5, **dadurch gekennzeichnet, daß** bei Verwendung eines Alkohol/Wasser-Gemischs der Alkoholgehalt dieser Lösung 10 - 90 Gew.-%, bevorzugt 30 - 70 Gew.-% beträgt.

**7.** Verfahren nach Anspruch 5 und/oder 6, **dadurch gekennzeichnet, daß** der Alkohol Methanol, Ethanol, Isopropanol, Ethylenglykol, 1,2-Propandiol oder 1,3-Propandiol ist.

**8.** Verfahren nach einem oder mehreren der Ansprüche 1 bis 4, **dadurch gekennzeichnet, daß** die Oberflächennachvernetzungslösung in einem Verhältnis von 1-20 Gew.-%, insbesondere 2,5-15 Gew.-%, bezogen auf die Masse des Polymeren eingesetzt wird.

**9.** Wasserabsorbierende Polymere, hergestellt nach dem Verfahren nach einem oder mehreren der Ansprüche 1 bis 8.

**10.** Verwendung der nach dem Verfahren nach einem oder mehreren der Ansprüche 1 bis 8 hergestellten Polymere in Hygieneartikeln, Verpackungsmaterialien und in Nonwovens.

**Claims**

**1.** A process for the surface postcrosslinking of water-absorbing polymers by treating the polymers with a surface postcrosslinking solution, the polymers being postcrosslinked and dried during or after the treatment by means of an increase in temperature, wherein the crosslinker contains an ester of boric acid having a dihydric or polyhydric alcohol dissolved in an inert solvent.

**2.** The process as claimed in claim 1, wherein the water-absorbing polymer is a polymeric acrylic acid or a polyacrylate, especially a polymeric acrylic acid or polyacrylate obtained by free-radical addition polymerization in the presence of a polyfunctional ethylenically unsaturated free-radical crosslinker which may additionally carry one or more free hydroxyl groups

**3.** The process as claimed in claim 1 or 2, wherein the catalyst used for crosslinking comprises an inorganic acid, its anhydride, an organic acid or its anhydride.

**4.** The process as claimed in claim 3, wherein the acid is boric, sulfuric, hydroiodic, phosphoric, tartaric, acetic or toluenesulfonic acid or the polymeric forms, anhydrides or acid salts thereof.

**5.** The process as claimed in one or more of claims 1 to 4, wherein the inert solvent is water, a mixture of water with organic solvents of unlimited solubility in water, or a mixture of water with monohydric or polyhydric alcohols.

**6.** The process as claimed in claim 5, wherein if an alcohol/water mixture is used the alcohol content of this solution is 10-90% by weight, preferably 30-70% by weight.

**7.** The process as claimed in claim 5 or 6, wherein the alcohol is methanol, ethanol, isopropanol, ethylene glycol,

1,2-propanediol or 1,3-propanediol.

**8.** The process as claimed in one or more of claims 1 to 4, wherein the surface postcrosslinking solution is employed in a proportion of 1-20% by weight, in particular 2.5-15% by weight, based on the mass of the polymer.

**9.** A water-absorbing polymer prepared by the process as claimed in one or more of claims 1 to 8.

**10.** The use of a polymer prepared by the process as claimed in one or more of claims 1 to 8 in a hygiene article, packaging material or nonwoven.

**Revendications**

**1.** Procédé de réticulation superficielle de polymères absorbant l'eau par traitement des polymères au moyen d'une solution de post-réticulation de surface, où les polymères sont post-réticulés et séchés pendant ou après le traitement par une élévation de température, **caractérisé en ce que** l'agent réticulant contient un ester d'acide borique avec un alcool bivalent ou polyvalent dans un solvant inerte.

**2.** Procédé selon la revendication 1, **caractérisé en ce que** le polymère absorbant l'eau est un polymère d'acide acrylique ou un polyacrylate, en particulier un polymère d'acide acrylique ou un polyacrylate obtenus par polymérisation radicalaire en présence d'un réticulant radicalaire éthyléniquement insaturé polyfonctionnel, qui peut en outre porter encore un ou plusieurs radicaux hydroxyle libres.

**3.** Procédé selon la revendication 1 et/ou 2, **caractérisé en ce que** l'on utilise pour la réticulation un catalyseur comportant un acide inorganique, son anhydride correspondant, un acide organique ou son anhydride correspondant.

**4.** Procédé selon la revendication 3, **caractérisé en ce que** les acides sont de l'acide borique, de l'acide sulfurique, de l'acide iodhydrique, de l'acide phosphorique, de l'acide tartrique, de l'acide acétique, de l'acide toluène-sulfonique, ainsi que leurs formes polymères, anhydrides ou sels acides.

**5.** Procédé selon l'une ou plusieurs des revendications 1 à 4, **caractérisé en ce que** le solvant inerte est de l'eau, un mélange d'eau et de solvants organiques à solubilité illimitée dans l'eau, ou un mélange d'eau et d'alcools monovalents ou polyvalents.

**6.** Procédé selon la revendication 5, **caractérisé en ce que**, lorsque l'on utilise un mélange alcool/eau, la teneur en alcool de cette solution est de 10 à 90% en poids, de préférence de 30 à 70% en poids.

**7.** Procédé selon la revendications et/ou 6, **caractérisé en ce que** l'alcool est du méthanol, de l'éthanol, de l'isopropanol, de l'éthylène glycol, du 1,2-propanediol ou du 1,3-propanediol.

**8.** Procédé selon une ou plusieurs des revendications 1 à 4, **caractérisé en ce que** la solution de post-réticulant de surface est mise en oeuvre en proportion de 1 à 20% en poids, en particulier de 2,5 à 15% en poids, par rapport à la masse du polymère.

**9.** Polymères absorbant l'eau, préparés par le procédé selon l'une ou plusieurs des revendications 1 à 8.

**10.** Utilisation des polymères préparés par le procédé selon l'une ou plusieurs des revendications 1 à 8 dans des articles d'hygiène, des matériaux d'emballage et des non tissés.